# EUROPEAN PATENT APPLICATION

(11) **EP 2 476 421 A1**
(43) Date of publication of application: **18.07.2012**
(21) Application number: 12150956.6
(22) Date of filing: 12.01.2012
(51) Int. Cl.: A61K 31/7016, A61K 31/728, A61P 1/04, A61P 17/02

(54) **Hyaluronic acid based mucoadhesive therapeutic composition for the prevention and treatment of epithelial and mucosal lesions of the human body**

(30) Priority: 17.01.2011 IT MI20110033
(71) Applicant: BIOFARMITALIA S.p.A., 20144 Milano (IT)
(72) Inventor: Di Schiena, Michele, 20087 Robecco sul Naviglio (MI) (IT)
(74) Representative: Frignoli, Luigi

(57) **Abstract**

A sucralfate and hyaluronic acid based mucoadhesive composition of very low viscosity and its physiologically acceptable salts for the treatment and prevention of epithelial and mucosal lesions of the human body.

## Description

The present invention relates to a sucralfate and hyaluronic acid based mucoadhesive composition of very low viscosity and its physiologically acceptable salts such as its sodium and potassium salts.

The composition is for human and animal use.

Sucralfate is the hydroxyaluminium complex of saccharose octosulfuric ester; the compound is practically insoluble in water and in the common organic solvents.

Sucralfate is known (see for example "The Merck Index") and is marketed for example by the German firm BK GIULINI GmbH.

Sucralfate has long been used in the prevention and treatment of gastric ulcer, duodenal ulcer, acute gastritis, symptomatic chronic gastritis, NSAID gastropathies, reflux esofage; compositions for this therapeutic use are marketed under various brand names (Martindale, The Complete Drug Reference, 34th edition).

In this use, sucralfate performs its therapeutic activity by forming a protective barrier on the gastric and duodenal mucosa, and also maintains the physiological mucosa pH value, an important factor for therapeutic purposes.

Sucralfate is also used in the gynecological field (in Italy as the brand name Alfa REPAGIN^{R}) for application to female external genitals.

Its use in odontostomatology as a palliative in oral mucositis is also known (The Merck Manual of Geriatrics).

The preparation of low viscosity hyaluronic acid is described in US 5,093,487 and US 4,517,295.

Very low viscosity hyaluronic acid and its physiologically acceptable salts, such as the sodium and potassium salt, is also known and is marketed under various brand names; for example as the registered trademark Renovhyal it is marketed by the French firm Soliance for use in the cosmetic field.

Its use in the treatment and prevention of lesions of the epithelial tissue and connective tissue is known and is claimed in EP1908457A (inventors Di Schiena et al.).

This patent application specifies that the term "very low viscosity" means that the hyaluronic acid and its physiologically acceptable salts such as the sodium and potassium salt have a viscosity of 0 (zero) mPa.s at a concentration of 1% w/v in water at 20°C and a viscosity of less than 10 mPa.s at a concentration of 6% w/v in water at 20°C.

These viscosity values obviously refer to the viscosity variation after the substance has been dissolved in water and hence indicate the viscosity variation relative to the viscosity of water.

An instrument used to determine said viscosity is the vibration viscosimeter SV10 of the A&D Company Ltd. - United Kingdom. Hyaluronic acid of very low viscosity also has high solubility in water; these characteristics, peculiar to determined uses, can however negatively influence its mucoadhesiveness, which may not be adequate for therapeutic use on those mucosas subject to mechanical actions and with a marked presence of aqueous biological liquids which influence the hyaluronic acid concentration by decreasing it; mucosas with these properties are for example gastric mucosa, esophageal mucosa, oral mucosa, vaginal mucosa, external mucosa of the female and male genitals.

It has now been surprisingly found that sucralfate imparts a marked mucoadhesive property to very low viscosity hyaluronic acid and its physiologically acceptable salts such as the sodium salt and potassium salt.

This marked mucoadhesive property imparted by sucralfate to very low viscosity hyaluronic acid and its physiologically acceptable salts is favoured and incremented by the presence of water, such as that physiologically present in the mucus which covers mucosas and is naturally further favoured in those compositions in which water is used for administration for the scheduled therapeutic purpose.

To achieve this special marked mucoadhesiveness of very low viscosity hyaluronic acid, the sucralfate concentration is between 50% w/w and 150% w/w on the very low viscosity hyaluronic acid and its physiologically acceptable salts; the sucralfate concentration is preferably between 90% w/w and 110% w/w on the very low viscosity hyaluronic acid and its physiologically acceptable salts such as the sodium and potassium salts. The sucralfate concentration can naturally be strongly increased in those compositions in which the sucralfate is also used for its already known properties, such as that of protective barrier for mucosal tissue and of maintaining mucosa physiological pH such as in the case of gastric mucosa, vaginal mucosa, oral mucosa.

Physiologically acceptable excipients compatible with very low viscosity hyaluronic acid, with sucralfate and with the scheduled use can also be added to the composition of the present invention.

Preferred excipients are chosen from the group consisting of alcohols, natural and synthetic oils, gelling products, suspendants, emulsifiers or thickeners, inert powders, natural and synthetic polymers, synthetic and natural sweeteners, synthetic and natural fragrances, synthetic and natural perfumes, synthetic and natural dyes, synthetic and natural preservatives; compounds favouring epithelial and connective absorption can also be used.

The composition of the present invention also finds useful application in various therapeutic sectors, such as in gastroenterology, gynecology, odontostomatology.

The forms in which the composition of the present invention is preferably used are all those already known in the art, such as compositions in gels, pastes, suspensions including in situ, mouthwashes including spray form, tablets, capsules, pessaries and vaginal suppositories. creams including spray forms, mucoadhesive film commonly known as "film food", particularly useful in treating oral aphthas and particularly useful in recurring aphthas.

Some non-limiting examples of implementation of the invention will now be described.

### EXAMPLE 1

### Base composition

### Formulation A

| | |
|---|---|
| Sucralfate | 500.00 g |
| very low viscosity sodium hyaluronate | 500.00 g |

### Formulation B

| | |
|---|---|
| Sucralfate | 450.00 g |
| very low viscosity sodium hyaluronate | 500.00 g |

### Formulation C

| | |
|---|---|
| sucralfate | 550.00 g |
| very low viscosity sodium hyaluronate | 550.00 g |

The ingredients of the three aforestated formulations are mixed carefully, the resultant compositions being used at the concentrations stated in the following Examples.

Further sucralfate can be added as such at very high concentrations to these compositions and to the compositions of the following Examples in order to utilize its known properties, such as its protective properties, its barrier properties and for maintenance of mucosa physiological pH.

### EXAMPLE 2

### Composition in single dose sachet for use in gastroenterology

| | |
|---|---|
| composition of Formulation A of Example 1 | 0.010 g |
| silicon dioxide | 0.010 g |
| sweetener | 0.100 g |
| Maltodextrin to make up to 3 g | 1.980 g |

This composition (to which one or more fragrances can be added) is administered orally by suspending it in water for the prevention and treatment of lesions of the gastroesophageal mucosa.

### EXAMPLE 3

### Single dose liquid composition for use in gastroenterology

| | |
|---|---|
| Composition of Formulation B of Example 1 | 0.025 g |
| Fructose | 3.000 g |
| Sorbic acid | 0.010 g |
| Sodium benzoate | 0.010 g |
| Strawberry flavouring | 0.020 g |
| Cochineal carmine dye | 0.005 g |
| Purified water | 16.930 g |

### EXAMPLE 4

### Gel composition for use in odontostomatology

| | |
|---|---|
| Composition of Formulation C of Example 1 | 0.100 g |
| Allantoin | 0.150 g |
| Glycerol | 10.000 g |
| 95° ethanol | 4.000 g |
| Sodium benzoate | 0.100 g |
| Purified water | 85.650 g |

This composition (which may be added with one colorant or one or more aromas) is useful for the treatment of oral mucositis, recurrent aphtous stomatitis.

### EXAMPLE 5

### Gel composition for use in gynecology (protection and treatment of vaginal and vulvar mucosa)

| | |
|---|---|
| Composition of Formulation A of Example 1 | 0.050 g |
| Carbopol 940 | 0.800 g |
| Propylene glycol | 5.000 g |
| Sodium EDTA | 0.200 g |
| Nipagin | 0.140 g |
| Nipasol | 0.020 g |
| Purified water | 93.790 g |

This composition can contain a sodium hydroxide solution at 100 g/l concentration.

### EXAMPLE 6

### Ovule composition for use in gynecology (vaginal mucosa)

| | |
|---|---|
| Composition of Formulation A of Example 1 | 0.030 g |
| Animal gelatine | 0.510 g |
| Glycerol | 1.830 g |
| Purified water | 0.630 g |

### EXAMPLE 7

### Cream composition for protection and treatment of female and male external mucosas

| | |
|---|---|
| Composition of Formulation A of Example 1 | 0.400 g |
| Bisabolol | 0.050 g |
| Wheat germ oil | 5.000 g |
| Sodium benzoate | 0.100 g |
| Sorbic acid | 0.050 g |
| Boric acid | 0.050 g |
| Salcare SC91 (registered trademark) | 3.000 g |
| Purified water | 91.350 g |

In the above illustrated examples the component "sucralfate" has merely been stated as such, however it should be noted that said sucralfate is preferably used in micronized form.

## Claims

1. A mucoadhesive therapeutic composition for the prevention and treatment of epithelial and mucal lesions of the human body, **characterised by** comprising hyaluronic acid or its physiologically acceptable salts and sucralfate between 50% and 150% by weight on the weight of said hyaluronic acid or its salts, said hyaluronic acid or its salts being of low viscosity type, of less than 10 mPa.s at a concentration of 6% in water at 20°C.

2. A composition as claimed in claim 1, **characterised in that** the sucralfate is present in the composition at a percentage between 90% and 110% by weight on the weight of the hyaluronic acid or its salts.

3. A composition as claimed in claims 1 and 2, **characterised by** comprising at least one excipient chosen from the group comprising preferred excipients chosen from the group consisting of alcohols, natural and synthetic oils, gelling products, suspendants, emulsifiers or thickeners, inert powders, natural and synthetic polymers, synthetic and natural sweeteners, synthetic and natural fragrances, synthetic and natural perfumes, synthetic and natural dyes, synthetic and natural preservatives (at the concentrations envisaged by individual states).
